# FASCICULE DE BREVET EUROPEEN

(11) **EP 4 450 094 B1**
(45) Date de publication et mention de la délivrance du brevet: **02.09.2026**
(21) Numéro de dépôt: 24169699.6
(22) Date de dépôt: 11.04.2024
(51) Int. Cl.: A61L 2/18, A61L 2/22, B25J 1/02, B25J 1/12, B25J 5/00, B25J 19/02

(54) **DISPOSITIF DE DÉCONTAMINATION DE SURFACES EN HAUTEUR, POUR DÉCONTAMINER NOTAMMENT UNE SURFACE DE TOIT D'UN VÉHICULE MILITAIRE, ET VÉHICULE DE DÉCONTAMINATION LE COMPRENANT**
VORRICHTUNG ZUR DEKONTAMINATION VON OBERFLÄCHEN IN DER HÖHE, INSBESONDERE ZUR DEKONTAMINATION EINER DACHFLÄCHE EINES MILITÄRISCHEN FAHRZEUGS, UND DEKONTAMINATIONSFAHRZEUG DAMIT
DEVICE FOR DECONTAMINATING SURFACES AT HEIGHT, IN PARTICULAR FOR DECONTAMINATING A ROOF SURFACE OF A MILITARY VEHICLE, AND DECONTAMINATION VEHICLE COMPRISING SAME

(30) Priorité: 19.04.2023 FR 2303825
(43) Date de publication de la demande: 23.10.2024
(73) Titulaire: KNDS CBRN, 78034 Versailles Cedex (FR)
(72) Inventeur: BAPTISTA, Daniel, 78034 VERSAILLES CEDEX (FR); JANDET, Fabrice, 78034 VERSAILLES CEDEX (FR); SERRANO, Joris, 78034 VERSAILLES CEDEX (FR)
(74) Mandataire: Cabinet Chaillot

(56) Documents cités:
- CN-A- 113 499 903
- CN-U- 215 607 919
- DE-U1- 202020 105 606

## Description

Le domaine technique de l'invention est celui de la décontamination, et plus spécifiquement, celui de la décontamination des véhicules terrestres ou aériens ou autres engins, matériels et infrastructures exposés à des agents contaminants nucléaires, radioactifs, biologiques et/ou chimiques (NRBC).

Une exposition à de tels agents contaminants peut résulter, par exemple, de l'utilisation d'armes nucléaires, de bombes à base de substances radioactives, d'armes biologiques permettant la dispersion de virus, de bactéries ou de toxines ou de munitions chimiques déployées par obus, roquettes, missiles ou épandages. Cette exposition peut également résulter d'événements involontaires, tels que des défaillances accidentelles d'installations industrielles nucléaires ou chimiques.

La décontamination consiste à rétablir la sécurité en absorbant, détruisant, neutralisant, rendant inoffensif ou éliminant les agents chimiques ou biologiques ou en enlevant les matières radioactives, les recouvrant ou les entourant.

Habituellement, la décontamination est réalisée en trois étapes : un pré-traitement, qui comprend un décrottage visant à enlever les matières labiles, un traitement, qui comprend une application d'un produit neutralisant et/ou facilitant le déplacement, et un post-traitement, qui comprend un rinçage permettant un enlèvement final des agents toxiques neutralisés. Le pré-traitement et le post-traitement sont réalisés avec de l'eau ou éventuellement avec un détergent, le traitement étant quant à lui réalisé avec des solutions chimiques.

La décontamination devrait se faire dès que possible après la contamination et devrait se dérouler aussi près que possible du point de contamination.

La présente invention concerne un dispositif de décontamination et un véhicule de décontamination comprenant un tel dispositif.

Le dispositif de décontamination et le véhicule de décontamination selon la présente invention sont particulièrement, mais non limitativement, adaptés à la décontamination d'un véhicule militaire, qu'il soit terrestre ou aérien, comme par un exemple un avion ou un hélicoptère, et plus particulièrement, à la décontamination d'une surface de toit d'un véhicule militaire.

La présente invention pourrait également être utilisée pour la décontamination de tout autre engin, ainsi que d'infrastructures et de matériels présents sur le lieu d'un événement NRBC, en particulier, les toits et éventuellement les façades des bâtiments et les surfaces situées en hauteur de matériels volumineux.

Actuellement, dans le domaine militaire, les véhicules tels que les voitures, les camions, les chars et les aéronefs, sont décontaminés par application **d'un** produit décontaminant sur la totalité de la surface via une lance. Afin de traiter toutes les surfaces, y compris les surfaces en hauteur, une nacelle et des moyens d'élévation de cette nacelle sont utilisés.

Plus particulièrement, comme divulgué dans le brevet européen EP1522454, la décontamination du dessus des véhicules militaires à décontaminer est usuellement réalisée au moyen d'une nacelle dans laquelle prend place un opérateur équipé d'une lance. La nacelle est située au bout d'un bras de grue et placée en surplomb du véhicule à décontaminer.

Cependant, l'utilisation **d'une** telle nacelle présente un grand nombre d'inconvénients. Tout d'abord, cette nacelle implique un travail en hauteur et expose donc l'opérateur à des risques de chute. Ces risques de sécurité pour l'opérateur sont augmentés dans un contexte de stress élevé, tel qu'un contexte de guerre ou d'exposition à des substances pathogènes. Pour réduire ces risques de sécurité, un entretien périodique et rigoureux de la nacelle et de ses moyens d'élévation est nécessaire. Or, un tel entretien réduit la disponibilité technique du matériel. En outre, pour réduire ces risques de sécurité, les opérateurs doivent être formés et habilités pour le travail en hauteur. Enfin, l'utilisation de telles nacelles suppose un temps de déploiement conséquent, ce qui augmente le délai d'intervention et la durée des opérations de décontamination. En outre, l'utilisation d'une telle nacelle implique que l'opérateur doit maintenir la lance, ce qui est pénible et occasionne de la fatigue pour l'opérateur.

On peut également citer le modèle d'utilité allemand DE 20 2020 105 606 U1 qui divulgue un dispositif de décontamination comprenant un mât portant un ensemble de décontamination, la hauteur de l'ensemble de décontamination pouvant être commandée manuellement par un utilisateur à l'aide de poignées reliées au mât. On connaît également la demande de brevet chinois CN 113 499 903 A qui divulgue un bras mécanique de désinfection dont la commande est automatique ou à distance. Enfin, le brevet chinois CN 215 607 919 U divulgue un dispositif de désinfection de l'intérieur d'un véhicule comprenant un bras manipulateur fixé à un caisson mobile, un écran de commande du dispositif étant positionné sur le caisson mobile.

La Société déposante a donc cherché à proposer une solution de décontamination de surfaces en hauteur, applicable notamment à une surface de toit d'un véhicule militaire, facile et rapide à mettre en œuvre, y compris dans des conditions difficiles telles que des conditions de guerre, et permettant d'éviter les risques et les inconvénients liés au travail en hauteur.

La présente invention a donc pour objet un dispositif de décontamination de surfaces en hauteur, pour décontaminer notamment une surface de toit d'un véhicule militaire soumis à une contamination, lequel dispositif comprend : - un mât s'étendant selon un axe longitudinal et ayant une première extrémité destinée à être fixée à une structure support et une seconde extrémité opposée ; - un ensemble bras monté en porte-à-faux au niveau de la seconde extrémité du mât, perpendiculairement à l'axe longitudinal du mât, et monté à rotation libre autour de l'axe longitudinal du mât ; - un ensemble rampe de décontamination monté à l'extrémité en porte-à-faux de l'ensemble bras et à rotation libre autour d'un axe parallèle à l'axe longitudinal du mât, l'ensemble rampe de décontamination comprenant au moins une rampe destinée à être reliée à une unité d'alimentation en produit décontaminant, ladite au moins une rampe s'étendant perpendiculairement à l'axe longitudinal du mât et étant munie de buses de diffusion disposées sur une face de la rampe, par exemple sur la face de la rampe côté première extrémité ; le dispositif étant caractérisé par le fait qu'il comprend en outre une perche de commande configurée pour être accessible par un opérateur au sol et accouplée à l'ensemble rampe de décontamination de telle sorte qu'une manipulation de la perche de commande par un opérateur au sol permet de commander les rotations de l'ensemble bras et de l'ensemble rampe de décontamination autour de leurs axes de rotation libre.

La perche de commande permet d'assurer la liaison entre un opérateur au sol, l'ensemble bras et l'ensemble rampe de décontamination. L'agencement de l'ensemble bras de manière déportée par rapport au mât, ainsi que le montage à rotation libre de l'ensemble bras par rapport au mât et de l'ensemble rampe de décontamination par rapport à l'ensemble bras, permettent à un opérateur de déplacer la rampe au-dessus de l'ensemble de la surface à décontaminer en évoluant au sol autour de ladite surface, notamment autour d'un véhicule à décontaminer. Ainsi, le dispositif selon l'invention permet à un opérateur de décontaminer une surface située en hauteur en restant debout au sol, sans utiliser de nacelle élévatrice portée par une grue ou de passerelle d'accès en hauteur.

Par rapport à l'état antérieur de la technique connu, le dispositif de décontamination selon la présente invention permet donc une mise en œuvre rapide et sécurisée et une amélioration de l'ergonomie de travail de l'opérateur.

En utilisation, lorsque le mât est en position verticale et que l'ensemble bras et la rampe s'étendent au-dessus d'une surface à décontaminer, avec les buses de diffusion tournées vers le sol, une manipulation de la perche de commande par un opérateur au sol permet de commander les rotations de l'ensemble bras et de la rampe autour d'axes verticaux. En particulier, en raison du montage à rotation libre de l'ensemble rampe de décontamination, un mouvement de rotation de la perche de commande permet de commander un mouvement de balayage de la rampe dans un plan horizontal au-dessus de la surface à décontaminer. Ce mouvement de balayage, associé au déplacement en rotation de l'ensemble bras dans un plan horizontal, permet de déplacer la rampe par rapport à la surface à décontaminer de façon à placer les buses en diffusion en regard de l'ensemble de ladite surface à décontaminer.

De préférence, l'ensemble bras comprend au moins deux bras manipulateurs articulés entre eux, à savoir au moins un bras manipulateur proximal articulé au mât et un bras manipulateur distal articulé au bras manipulateur proximal par une liaison pivot libre d'axe parallèle à l'axe longitudinal du mât et portant l'ensemble rampe de décontamination.

L'ensemble bras peut comprendre un bras manipulateur intermédiaire articulé entre le bras manipulateur proximal et le bras manipulateur distal par des liaisons pivot libre d'axes parallèles à l'axe longitudinal du mât.

De préférence, la liaison pivot libre entre deux bras manipulateurs articulés entre eux est configurée pour autoriser le pivotement des bras entre une position rabattue, dans laquelle les axes longitudinaux des bras forment un angle nul ou quasi-nul, et une position d'utilisation, dans laquelle les axes longitudinaux des bras forment des angles non nuls.

Dans un mode de réalisation particulier, l'ensemble rampe de décontamination comprend un bras porte-rampe portant la rampe, le bras porte-rampe s'étendant selon un axe parallèle à l'axe longitudinal du mât, la liaison pivot libre entre l'ensemble bras et l'ensemble rampe de décontamination étant une liaison pivot autour de l'axe longitudinal du bras porte-rampe, la perche de commande étant reliée solidaire en rotation au bras porte-rampe et s'étendant selon un axe coaxial à l'axe longitudinal du bras porte-rampe.

Avantageusement, afin de permettre d'ajuster la hauteur de la rampe, la rampe est montée mobile en translation le long du bras porte-rampe par une liaison glissière avec le bras porte-rampe.

L'ensemble rampe de décontamination pourrait également comprendre une rampe s'étendant parallèlement à l'axe longitudinal du mât et munie de buses de diffusion orientées perpendiculairement à l'axe longitudinal du mât. En utilisation, une telle rampe est une rampe verticale dont les buses sont aptes à disperser un produit décontaminant latéralement.

En variante, la rampe est montée mobile en rotation autour d'un axe de rotation qui est perpendiculaire à la fois à l'axe longitudinal du bras porte-rampe et à un axe longitudinal de la rampe, et/ou autour de son axe longitudinal. Cette mobilité en rotation peut être assurée par des liaisons pivots classiques, par exemple à indexage de position, bien connues en soi, permettant par exemple de placer la rampe dans une orientation horizontale pour la décontamination du dessus ou du dessous d'un véhicule et une orientation verticale pour la décontamination des parois verticales d'un véhicule, et de placer la rampe dans une orientation horizontale avec buses dirigées vers le bas pour la décontamination du dessus d'un véhicule et une orientation horizontale avec buses dirigées vers le haut pour la décontamination du dessous d'un véhicule.

De préférence, le dispositif comprend en outre une unité de surveillance comprenant au moins une caméra agencée de manière à être apte à viser une zone vers laquelle sont tournées les buses de diffusion et un écran apte à afficher l'image de la caméra. Cette unité de surveillance permet à un opérateur, évoluant au sol autour du véhicule à décontaminer, d'observer la zone de diffusion des buses de diffusion.

Avantageusement, l'unité de surveillance comprend en outre des buses de projection d'air agencées au niveau de la caméra afin de limiter des projections issues des buses de diffusion sur la caméra.

De préférence, la caméra est montée sur le bras porte-rampe.

Avantageusement, la perche de commande est montée de manière télescopique dans l'ensemble rampe de décontamination, le cas échéant dans le bras porte-rampe.

La perche de commande peut comporter un manche couplé à l'ensemble rampe de décontamination et une poignée radiale au niveau de l'extrémité libre du manche.

De préférence, la première extrémité du mât est articulée sur une embase par un axe de pivotement orthogonal à l'axe longitudinal du mât, un actionneur couplé au mât et destiné à être fixé à une structure support à laquelle est assujettie l'embase permettant un mouvement de pivotement du mât autour dudit axe de pivotement entre une position d'utilisation verticale et une position de rangement horizontale.

La présente invention a également pour objet un véhicule de décontamination de surfaces en hauteur, pour décontaminer notamment une surface de toit d'un véhicule militaire soumis à une contamination, le véhicule étant caractérisé par le fait qu'il comprend :
- un châssis mobile ;
- une unité d'alimentation en produit décontaminant supportée par le châssis mobile ; et
- un dispositif de décontamination tel que défini ci-dessus, le mât étant assujetti audit châssis mobile ou à ladite unité d'alimentation en produit décontaminant, la rampe étant reliée fluidiquement à l'unité d'alimentation en produit décontaminant par un chemin fluidique s'étendant le long du mât, puis le long de l'ensemble bras. Un tel chemin permet d'assurer la liaison fluidique dans les différentes positions des bras tout en permettant à l'opérateur au sol d'effectuer le tour de la surface à décontaminer sans être gêné par divers tuyaux ou câbles et sans avoir à manipuler de tels tuyaux ou câbles.

Un tel véhicule de décontamination peut être déplacé facilement au niveau de la surface à décontaminer, peut être installé sur tous types de terrains, peut fonctionner de manière autonome et est facile à mettre en œuvre, permettant ainsi de débuter des opérations de décontamination dans les plus brefs délais.

L'unité d'alimentation en produit décontaminant peut comprendre une source d'électricité, une source de produit décontaminant, ainsi qu'avantageusement des moyens de mise sous pression du produit décontaminant. On entend ici par l'expression « produit décontaminant » tout produit participant à la décontamination, comme par exemple de l'eau, des détergents, des solutions chimiques de décontamination, de sorte qu'un opérateur est en mesure de mettre en œuvre avec le véhicule de décontamination les étapes habituelles de pré-traitement, de traitement et de post-traitement. Les moyens de mise sous pression pourront être une source d'air comprimé. Un véhicule équipé d'une telle unité présente une autonomie complète, tant du point de vue énergétique que des éléments qu'il utilise, et peut donc être utilisé sur des sites isolés dépourvus de toutes sources d'énergie et d'éléments, par exemple d'eau, nécessaires à son fonctionnement.

Le véhicule de décontamination peut être un véhicule porteur tel qu'un camion ou un véhicule de type militaire.

Pour mieux illustrer l'objet de la présente invention, on va en décrire ci-après un mode de réalisation particulier, avec référence aux dessins annexés. Sur ces dessins :
[Fig. 1] est une vue schématique de côté d'un véhicule de décontamination équipé d'un dispositif de décontamination selon la présente invention, lequel dispositif de décontamination est représenté dans une position déployée et appliqué à la décontamination de la surface de toit d'un véhicule ;
[Fig. 2A] est une vue schématique de dessus du véhicule de décontamination de la Figure 1, représentant le dispositif de décontamination dans une première position d'utilisation ;
[Fig. 2B] est une vue analogue à celle de la Figure 2A, représentant le dispositif de décontamination dans une deuxième position d'utilisation ;
[Fig. 2C] est une vue analogue à celle des Figures 2A et 2B, représentant le dispositif de décontamination dans une troisième position d'utilisation ;
[Fig. 3A] est une vue schématique de côté du véhicule de décontamination de la Figure 1, illustrant une première phase du repli du dispositif de décontamination vers une position rangée ;
[Fig. 3B] est une vue analogue à celle de la Figure 3A, illustrant une deuxième phase du repli du dispositif de décontamination ;
[Fig. 3C] est une vue analogue à celles des Figures 3A et 3B, illustrant une troisième phase du repli du dispositif de décontamination ; et
[Fig. 3D] est une vue analogue à celles des Figures 3A, 3B et 3C, illustrant le dispositif de décontamination en position rangée.

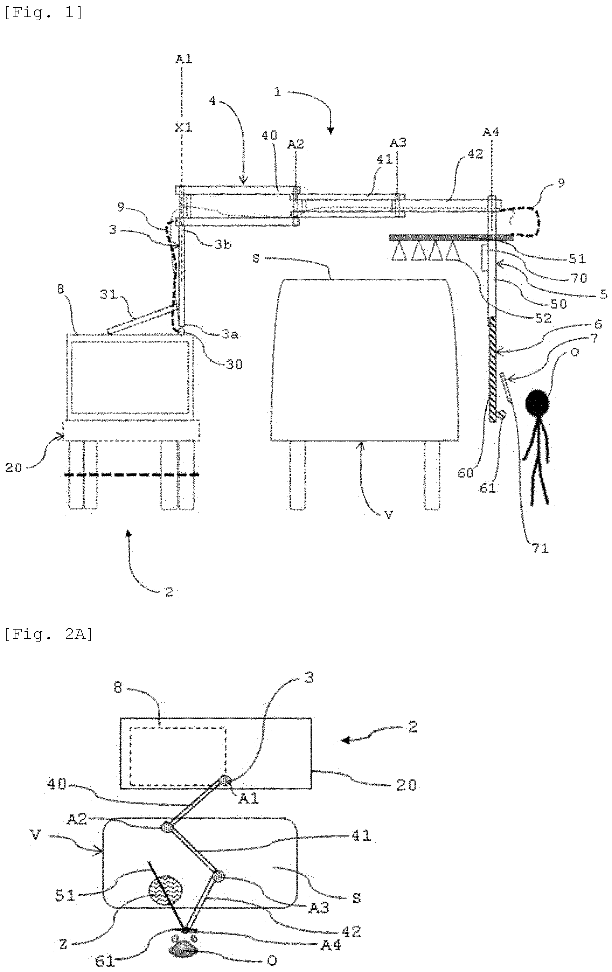

Le dispositif de décontamination 1 selon la présente invention est destiné à être monté sur un véhicule de décontamination 2 et agencé et configuré pour la décontamination de surfaces en hauteur. Dans le mode de réalisation représenté de manière schématique sur les Figures 1 et 2A à 2C, le dispositif de décontamination 1 est appliqué à la décontamination de la surface de toit S d'un véhicule militaire V. Le véhicule miliaire V à décontaminer peut être aussi bien un véhicule léger, qu'un camion ou un char. Le dispositif de décontamination 1 selon l'invention pourrait être utilisé pour la décontamination de tout autre engin servant de moyen de transport sur un site de guerre, d'une infrastructure militaire ou d'un matériel de gros volume utilisé par le personnel militaire. Le dispositif de décontamination 1 selon l'invention est particulièrement adapté pour la décontamination d'un véhicule militaire V dont la hauteur est élevée et ne permet à un opérateur O au sol équipé d'une lance classique de décontaminer la surface de toit S.

Si l'on se réfère à la Figure 1, on peut voir que le dispositif de décontamination 1 selon le mode de réalisation préféré de la présente invention comprend un mât 3, un ensemble bras 4, un ensemble rampe de décontamination 5, une perche de commande 6 et une unité de surveillance 7.

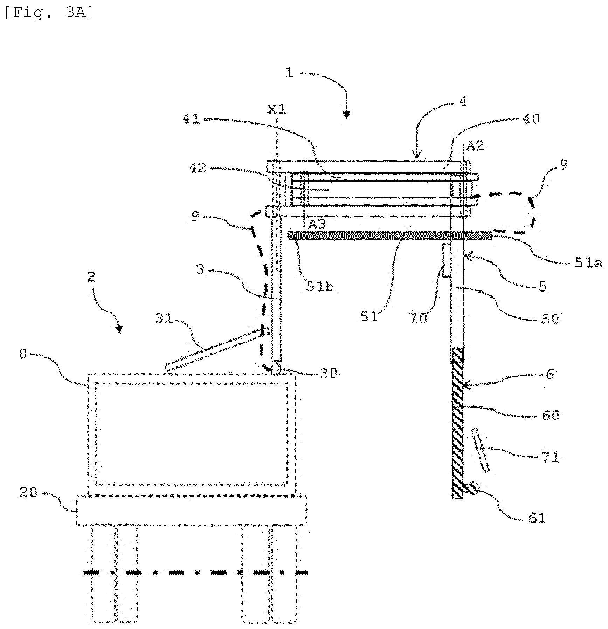

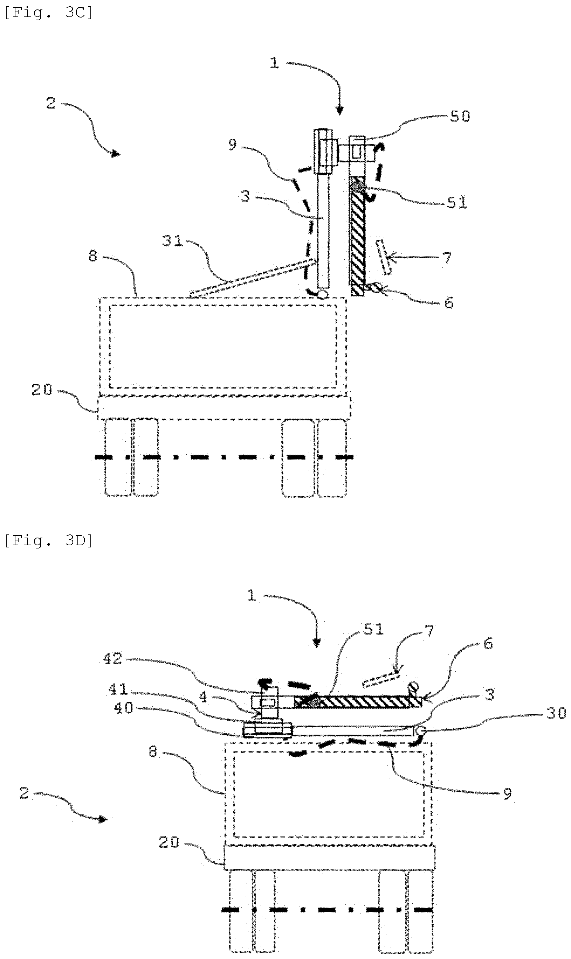

Le mât 3 s'étend selon un axe longitudinal X1 entre une première extrémité 3a et une seconde extrémité 3b opposée. Comme on peut le voir sur les Figures 1 et 3A à 3D, la première extrémité 3a du mât 3 est destinée à être fixée à une structure support portée par le véhicule de décontamination 2. Pour cela, une embase 30 est interposée entre le mât 3 et ladite structure support et est rendue solidaire de celle-ci. L'embase 30 comporte un axe de pivotement agencé pour être un axe de pivotement horizontal. La première extrémité 3a du mât 3 est montée à rotation autour de cet axe de pivotement horizontal, l'axe longitudinal X1 du mât 3 étant orthogonal à cet axe de pivotement horizontal. Ainsi, le mât 3 est monté mobile en rotation autour de l'axe de pivotement horizontal et est apte à basculer entre une position d'utilisation verticale et une position de rangement horizontale par rapport au sol. Le déplacement du mât 3 entre les positions verticale et horizontale est commandé par un actionneur 31 relié d'une part au mât 3 et d'autre part à la structure support dont l'embase 30 est solidaire. En position d'utilisation verticale (Figure 1), le mât 3 s'étend verticalement au-dessus de la structure support, avec la seconde extrémité 3b située à une certaine hauteur, en particulier à une hauteur supérieure à celle du véhicule V à décontaminer. En position de rangement horizontale (Figure 3D), le mât 3 est positionné horizontalement contre ou au voisinage de la structure support, et occupe donc le plus faible encombrement possible.

L'ensemble bras 4, en utilisation, est destiné à être suspendu au-dessus du véhicule V à décontaminer et est apte à occuper une pluralité de positions de support de l'ensemble rampe de décontamination 5.

Dans le mode de réalisation préféré représenté, l'ensemble bras 4 est un ensemble articulé comprenant un bras manipulateur proximal 40, un bras manipulateur intermédiaire 41 et un bras manipulateur distal 42. Il convient de souligner que le nombre de bras manipulateurs n'est pas limité à trois bras articulés et pourrait être inférieur ou supérieur à ce nombre. Toutefois, un nombre de bras manipulateurs supérieur ou égal à trois permet de s'adapter davantage à diverses dimensions de surfaces en hauteur à décontaminer. Chaque bras manipulateur 40, 41, 42 est un bras longitudinal ayant une première extrémité et une seconde extrémité opposée.

Le bras manipulateur proximal 40 a une première extrémité articulée sur le mât 3, au niveau de la seconde extrémité 3b du mât 3, autour d'un premier axe de pivot A1 coaxial ou parallèle à l'axe longitudinal X1 du mât 3. La liaison pivot entre le bras manipulateur proximal 40 et le mât 3 est une liaison pivot libre. Le bras manipulateur intermédiaire 41 a une première extrémité articulée à la seconde extrémité du bras manipulateur proximal 40 autour d'un deuxième axe de pivot A2 parallèle au premier axe de pivot A1 et à l'axe longitudinal X1 du mât 3. La liaison pivot entre le bras manipulateur intermédiaire 41 et le bras manipulateur proximal 40 est une liaison pivot libre. Le bras manipulateur distal 42 a une première extrémité articulée à la seconde extrémité du bras manipulateur intermédiaire 41 autour d'un troisième axe de pivot A3 parallèle aux premier A1 et deuxième A2 axes de pivot et à l'axe longitudinal X1 du mât 3. La liaison pivot entre le bras manipulateur distal 42 et le bras manipulateur intermédiaire 41 est une liaison pivot libre. La seconde extrémité du bras manipulateur distal 42, dite extrémité en porte-à-faux de l'ensemble bras 4, porte l'ensemble rampe de décontamination 5.

Les trois bras manipulateurs 40, 41, 42 ont leurs axes longitudinaux qui sont situés dans un même plan orthogonal à l'axe longitudinal X1 du mât 3 et aux premier à troisième axes de pivot A1, A2, A3. Ainsi, en utilisation, lorsque le mât 3 est en position d'utilisation verticale, l'ensemble bras 4 articulé est agencé dans un plan horizontal avec son extrémité en porte-à-faux déportée latéralement par rapport au mât 3 et est apte à occuper une pluralité de positions dans ce plan horizontal en fonction des mouvements de rotation des bras manipulateurs 40, 41, 42 autour de leurs axes de pivot verticaux A1, A2, A3.

Avantageusement, chaque liaison pivot libre autorise le pivotement du bras manipulateur 40, 41, 42 associé sur 360 degrés autour de son axe de pivot A1, A2, A3. Ainsi, chaque bras manipulateur 40, 41, 42 est apte à être placé dans une position d'utilisation (Figures 2A à 2C), dans laquelle il forme un angle quelconque par rapport à un bras manipulateur articulé sur celui-ci, et dans une position rabattue (Figures 3A à 3C), dans laquelle les bras manipulateurs 40, 41, 42 sont repliés les uns contre les autres, les axes longitudinaux des bras manipulateurs 40, 41, 42 formant entre eux des angles nuls ou quasi-nuls.

Le nombre de bras manipulateurs ainsi que les dimensions, notamment les longueurs, de ces bras sont choisies de telle sorte que l'extrémité en porte-à-faux de l'ensemble bras 4 peut être positionnée en n'importe quelle position autour du véhicule V ou analogue à décontaminer tandis que l'extrémité de l'ensemble bras 4 reliée au mât 3 demeure dans une position fixe par rapport au véhicule V à décontaminer. Les différents bras manipulateurs 40, 41, 42 peuvent être de même longueur ou de différentes longueurs.

Chaque bras manipulateur 40, 41, 42 peut être un bras creux pourvu d'une cavité longitudinale traversante apte à recevoir l'ensemble des moyens de liaison entre l'ensemble rampe de décontamination 5 porté par l'ensemble bras 4 et le véhicule de décontamination 2. En variante, chaque bras manipulateur pourrait être un bras plein comportant sur sa périphérie des éléments de support ou de passage de ces moyens de liaison.

En variante, chaque liaison pivot libre pourrait être remplacée par une liaison pivot dont le mouvement de rotation est commandé par des moyens de commande agencés sur les bras manipulateurs 40, 41, 42. Par exemple, le déplacement et le positionnement des bras manipulateurs en regard du véhicule V à décontaminer pourrait être commandé par des vérins ou par tout autre moyen moteur approprié. Cependant, dans le cas de vérins par exemple, une filerie électrique est nécessaire pour l'actionnement. Le mode de réalisation préféré de l'invention, avec les liaisons pivots libres et la commande manuelle des mouvements de rotation des bras présente les avantages de ne pas nécessiter de pièces coûteuses de type vérin ou moteur, et d'être plus fiable qu'une version avec des moyens de commande susceptibles de dysfonctionner.

L'ensemble rampe de décontamination 5 est agencé pour permettre la pulvérisation ou la diffusion **d'un** produit décontaminant au-dessus de la totalité de la surface en hauteur S à décontaminer. A cet effet, l'ensemble rampe de décontamination 5 est destiné à être relié fluidiquement à une unité 8 d'alimentation en produit décontaminant portée par le véhicule de décontamination 2. Avantageusement, une telle unité 8 d'alimentation en produit décontaminant est pourvue de sources d'énergie, notamment d'une source d'électricité, et de l'ensemble des éléments nécessaires à la production d'un produit décontaminant, notamment d'un réservoir d'eau ou de liquide quelconque.

Dans le mode de réalisation préféré représenté, l'ensemble rampe de décontamination 5 comprend un bras porte-rampe 50 et une rampe 51.

Le bras porte-rampe 50 est monté au niveau de l'extrémité en porte-à-faux de l'ensemble bras 4, autrement dit au niveau de la seconde extrémité du bras manipulateur distal 42. Le bras porte-rampe 50 est un bras longitudinal ayant une première extrémité et une seconde extrémité opposée. La première extrémité est articulée sur le bras manipulateur distal 42 autour d'un quatrième axe de pivot A4 parallèle à l'axe longitudinal X1 du mât 3. La liaison pivot entre le bras porte-rampe 50 et le bras manipulateur distal 42 est une liaison pivot libre. Le quatrième axe de pivot A4 est coaxial à l'axe longitudinal du bras porte-rampe 50. Autrement dit, le bras porte-rampe 50 s'étend selon un axe parallèle à l'axe longitudinal X1 du mât 3. Ainsi, en utilisation, lorsque le mât 3 est en position d'utilisation verticale, le bras porte-rampe 50 s'étend verticalement et est apte à pivoter librement autour de son axe longitudinal vertical, la seconde extrémité du bras porte-rampe 50 s'étendant au-dessous de l'ensemble bras 4.

La rampe 51 est portée par le bras porte-rampe 50. Cette rampe 51 est une rampe longitudinale ayant une extrémité de raccordement 51a destinée à être raccordée fluidiquement à l'unité 8 d'alimentation en produit décontaminant et une extrémité libre 51b opposée. La rampe 51 comporte une pluralité de buses de diffusion 52 régulièrement espacées le long de celle-ci et disposées sur la face de la rampe 51 opposée à l'ensemble bras 4. La rampe 51 est reliée au bras porte-rampe 50 au voisinage de son extrémité de raccordement 51a, l'extrémité libre 51b étant déportée par rapport au bras porte-rampe 50. La rampe 51 s'étend perpendiculairement à l'axe longitudinal du bras porte-rampe 50 et donc perpendiculairement à l'axe longitudinal X1 du mât 3 et au quatrième axe de pivot A4. Ainsi, en utilisation, lorsque le mât 3 est en position d'utilisation verticale, la rampe 51 est située dans un plan horizontal et est apte à pivoter librement autour du quatrième axe de pivot vertical A4, ici avec les buses de diffusion 52 dirigées vers le sol. Cette rampe 51 dite rampe horizontale est montée mobile en translation le long du bras porte-rampe 50 entre les première et seconde extrémités du bras porte-rampe 50, donc entre l'ensemble bras 4 et la perche de commande 6. A cet effet, une liaison glissière est formée entre la rampe 51 et le bras porte-rampe 50. En utilisation, cette liaison glissière permet d'ajuster la hauteur de la rampe 51 afin de s'adapter à la hauteur de la surface S à décontaminer. Ainsi, la distance entre les buses de diffusion 52 et la surface S à traiter est ajustée manuellement par l'opérateur O au sol en fonction de la hauteur de la surface S et de paramètres liés à la diffusion du produit décontaminant, notamment de la pression du produit projeté.

L'ensemble rampe de décontamination 5 pourrait également comprendre une rampe dite rampe verticale s'étendant parallèlement à l'axe longitudinal X1 du mât 3 et munie de buses de diffusion orientées perpendiculairement à l'axe longitudinal du mât 3. En utilisation, une telle rampe permettrait de disperser un produit décontaminant latéralement, par exemple sur les parois latérales du véhicule V à décontaminer.

En variante, la rampe 51 est également reliée au bras porte-rampe 50 par une liaison pivot autorisant une rotation de la rampe 51 autour de l'axe longitudinale de cette dernière, pour pouvoir diriger les buses de diffusion 52 vers le haut ou vers le bas, et par une liaison pivot autorisant une rotation de la rampe 51 autour d'un axe de rotation perpendiculaire à l'axe longitudinal de la rampe 51 et à l'axe longitudinal du bras porte-rampe 50, pour pouvoir placer la rampe 51 à l'horizontale ou à la verticale. De cette manière, l'opérateur O, en commandant les différentes rotations de la rampe 51 et sa translation par rapport au bras porte-rampe 50, peut décontaminer le dessus, les parois latérales et le dessous du véhicule V.

La perche de commande 6, en utilisation, est destinée à être saisie par un opérateur O au sol. La perche de commande 6 est agencée pour permettre, en utilisation, une commande de la rotation de la rampe 51 dans un plan horizontal ainsi qu'une commande de la rotation de chaque bras manipulateur 40, 41, 42 autour de son axe de pivot vertical A1, A2, A3. En d'autres termes, la perche de commande 6 est configurée et agencée pour permettre la commande, depuis le sol, du déplacement de la rampe 51 au-dessus de la totalité de la surface S à décontaminer, sans difficulté et sans fatigue pour l'opérateur O.

La perche de commande 6 comporte un manche 60 et une poignée 61. Le manche 60 est un manche longitudinal ayant une première extrémité reliée au bras porte-rampe 50 et une seconde extrémité libre opposée. La liaison entre la perche de commande 6 et le bras porte-rampe 50 est telle que le bras porte-rampe 50 est solidaire en rotation de la perche de commande 6. Le manche 60 s'étend dans le prolongement du bras porte-rampe 50, l'axe longitudinal du manche 60 étant coaxial à l'axe longitudinal du bras porte-rampe 50. La poignée 61 est solidaire de la région d'extrémité libre du manche 60 et s'étend radialement par rapport au manche 60. De manière avantageuse, le bras porte-rampe 50 est un bras creux et le manche 60 de la perche de commande 6 est monté de manière télescopique dans le bras porte-rampe 50.

L'unité de surveillance 7 est agencée pour permettre à un opérateur O au sol, manipulant la perche de commande 6, d'observer la zone de diffusion Z des buses de diffusion 52, et donc de s'assurer que l'ensemble de la surface S à décontaminer est traitée par lesdites buses 52.

Dans le mode de réalisation préféré, représenté de manière schématique, l'unité de surveillance 7 comprend au moins une caméra 70 et un écran d'affichage 71. L'au moins une caméra 70 est positionnée au niveau du bras porte-rampe 50 et orientée de manière à être apte à prendre des images de la zone de diffusion Z des buses de diffusion 52. La caméra 70 peut être entourée de buses de projection d'air (non représentées) configurées pour empêcher le produit décontaminant issu des buses de diffusion 52 de recouvrir l'objectif de la caméra 70. L'écran 71 est apte à afficher les images provenant de l'au moins une caméra 70. De préférence, l'écran 71 est positionné au niveau de la perche de commande 6, côté poignée 61. Ainsi, un opérateur O manipulant la perche de commande 6 peut visualiser la zone Z de la surface S à décontaminer visée par les buses de diffusion 52 et positionner la rampe 51 et l'ensemble bras 4 de manière appropriée.

Le dispositif de décontamination 1 de surfaces en hauteur selon la présente invention est destiné à équiper un véhicule de décontamination 2. Ce véhicule de décontamination 2 est destiné à être déplacé jusqu'à une surface S devant être décontaminée, notamment à côté d'un véhicule militaire V ayant subi une contamination, et à permettre la mise en œuvre rapide et de manière autonome d'une opération de décontamination de ladite surface S. Le véhicule de décontamination 2 selon la présente invention comprend un châssis mobile 20 supportant au moins une unité 8 d'alimentation en produit décontaminant et au moins un dispositif de décontamination 1.

De préférence, le véhicule de décontamination 2 est un véhicule routier, tel qu'un camion ou un véhicule militaire.

Le châssis mobile 20 est un châssis monté sur roues, notamment sur des roues avant et des roues arrière. Ce châssis 20 comprend une plateforme de chargement et une cabine (non représentée) intégrant un poste de conduite.

L'au moins une unité 8 d'alimentation en produit décontaminant est supportée par la plateforme de chargement, et positionnée derrière la cabine. Cette unité 8 comporte un conteneur qui contient l'ensemble des équipements nécessaires à la décontamination, tels que des tuyaux, un réservoir d'eau, un groupe électrogène, une unité de nettoyage haute pression, etc.

Dans le mode de réalisation représenté, l'unité 8 d'alimentation en produit décontaminant, en particulier, le conteneur de cette unité 8, constitue la structure support à laquelle sont fixés l'embase 30 et l'actionneur 31 reliés au mât 3. Ainsi, le mât 3 s'étend au-dessus de la surface supérieure du conteneur. La solidarisation du dispositif de décontamination 1 au conteneur de l'unité 8 d'alimentation en produit décontaminant permet à l'ensemble bras 4 d'être suspendu à une hauteur élevée sans la nécessité d'une longueur de mât 3 importante. En variante, le mât 3 pourrait être relié à la plateforme du châssis 20, ce qui permettrait de réduite la hauteur globale du véhicule de décontamination 2.

Un chemin fluidique 9 est formé entre la rampe 51 et l'unité 8 d'alimentation en produit décontaminant. Ce chemin 9 comprend au moins une conduite s'étendant depuis un réservoir en produit décontaminant de l'unité 8 d'alimentation en produit décontaminant, le long du mât 3, le long de chaque bras manipulateur 40, 41, 42 et jusqu'à la rampe 51. La conduite peut être retenue le long des divers éléments du dispositif 1 par tout moyen approprié, notamment par des passages de conduite appropriés prévus le long du dispositif 1.

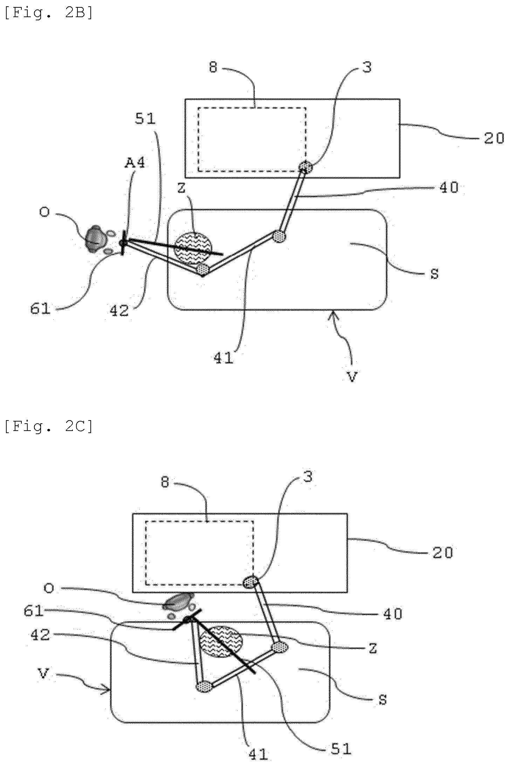

En utilisation, comme on peut le voir sur les Figures 1 et 2A à 2C, le dispositif de décontamination 1 est déployé au-dessus d'une surface S à décontaminer, notamment au-dessus d'une surface de toit S d'un véhicule militaire V. En position déployée d'utilisation, le mât 3 s'étend verticalement au-dessus du châssis 20 du véhicule de décontamination 2, l'ensemble bras 4 s'étend horizontalement au-dessus du véhicule militaire V à décontaminer et à une hauteur supérieure à la hauteur de la surface de toit S, et le bras porte-rampe 50 et la perche de commande 6 s'étendent verticalement d'un côté du véhicule V à décontaminer, la poignée 61 étant à hauteur d'homme. Un opérateur O au sol saisit la perche de commande 6 et se déplace autour du véhicule V à décontaminer tout en orientant la perche 6 et donc la rampe 51 afin que les buses 52 soient dirigées vers la surface de toit S. Sur les Figures 1 et 2A, l'opérateur O positionne le bras porte-rampe 50 du côté du véhicule V à décontaminer opposé au véhicule de décontamination 2. Sur la Figure 2B, l'opérateur O positionne le bras porte-rampe 50 du côté arrière du véhicule V à décontaminer. Sur la Figure 2C, l'opérateur O positionne le bras porte-rampe 50 entre le véhicule de décontamination 2 et le véhicule V à décontaminer. Dans chaque cas, les bras manipulateurs 40, 41, 42 se déplacent automatiquement au-dessus de la surface de toit S par rotation autour de leurs axes de pivot A1, A2, A3. Ainsi, l'opérateur O peut cheminer librement autour du véhicule V à décontaminer, sans aucune charge à soutenir, son seul rôle étant de positionner correctement la rampe 51 au-dessus de la totalité de la surface de toit S par une manipulation de la perche de commande 6. Par ailleurs, le dispositif 1 à l'état déployé n'empêche pas le travail d'un second opérateur au sol. Ainsi, un autre opérateur au sol peut diriger un produit décontaminant sur les parois latérales du véhicule V à décontaminer de manière simultanée à l'opération de décontamination de la surface de toit S.

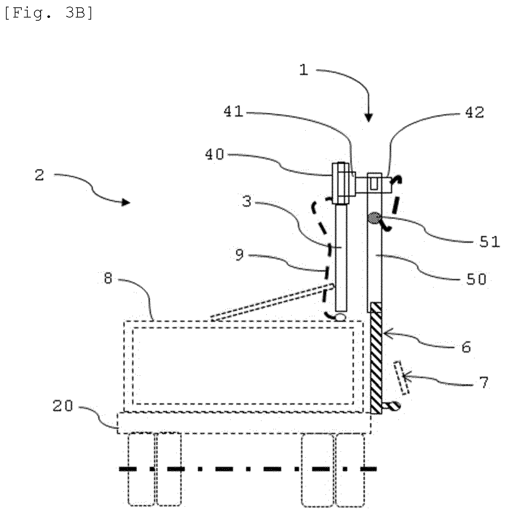

Comme on peut le voir sur les Figures 3A à 3D, une fois l'opération de décontamination terminée, le dispositif de décontamination 1 peut être replié dans une position rangée. Pour cela, comme on peut le voir sur la Figure 3A, l'ensemble bras 4 est tout d'abord replié. A cet effet, la perche de commande 6 est déplacée de manière à faire pivoter chaque bras manipulateur 40, 41, 42 autour de son axe de pivot A1, A2, A3 jusqu'à ce que le bras manipulateur distal 42 soit rabattu sous le bras manipulateur intermédiaire 41 et que le bras manipulateur intermédiaire 41 soit rabattu sous le bras manipulateur proximal 40. Puis, comme on peut le voir sur la Figure 3B, le bras manipulateur proximal 40, ainsi que la rampe 51, sont orientés parallèlement à l'axe longitudinal du châssis 20, par pivotement de 90 degrés autour de leurs axes de de pivot A1 et A4, respectivement. Ainsi, l'ensemble bras 4 n'est plus en porte-à-faux par rapport au châssis 20. Ensuite, comme on peut le voir sur la Figure 3C, la perche de commande 6 est déplacée par coulissement à l'intérieur du bras porte-rampe 50, jusqu'à ce que le manche 60 soit escamoté à l'intérieur du bras porte-rampe 50. Enfin, comme on peut le voir sur la Figure 3D, le mât 3 est basculé de sa position verticale à sa position horizontale par pivotement autour de l'axe de pivotement horizontal sous l'effet de l'actionneur 31. Une telle position rangée permet de réduire l'encombrement du véhicule de décontamination 2 et permet donc un transport aisé du dispositif de décontamination 1. Le passage de la position déployée à la position rangée, et inversement, est aisé et rapide à mettre en œuvre.

Il est bien entendu que les modes de réalisation particuliers qui viennent d'être décrits ont été donnés à titre indicatif et non limitatif, et que des modifications peuvent être apportées sans que l'on s'écarte pour autant du cadre de la présente invention.

## Revendications

1. - Dispositif de décontamination (1) de surfaces en hauteur, pour décontaminer notamment une surface de toit (S) d'un véhicule militaire (V) soumis à une contamination, lequel dispositif (1) comprend :
- un mât (3) s'étendant selon un axe longitudinal (X1) et ayant une première extrémité (3a) destinée à être fixée à une structure support et une seconde extrémité (3b) opposée ;
- un ensemble bras (4) monté en porte-à-faux au niveau de la seconde extrémité (3b) du mât (3), perpendiculairement à l'axe longitudinal (X1) du mât (3), et monté à rotation libre autour de l'axe longitudinal (X1) du mât (3) ;
- un ensemble rampe de décontamination (5) monté à l'extrémité en porte-à-faux de l'ensemble bras (4) et à rotation libre autour d'un axe (A4) parallèle à l'axe longitudinal (X1) du mât (3), l'ensemble rampe de décontamination (5) comprenant au moins une rampe (51) destinée à être reliée à une unité (8) d'alimentation en produit décontaminant, ladite au moins une rampe (51) s'étendant perpendiculairement à l'axe longitudinal (X1) du mât (3) et étant munie de buses de diffusion (52) disposées sur une face de la rampe (51) ;
le dispositif (1) étant **caractérisé par le fait qu'**il comprend en outre une perche de commande (6) configurée pour être accessible par un opérateur (O) au sol et accouplée à l'ensemble rampe de décontamination (5) de telle sorte qu'une manipulation de la perche de commande (6) par un opérateur (O) au sol permet de commander les rotations de l'ensemble bras (4) et de l'ensemble rampe de décontamination (5) autour de leurs axes (A1-A4) de rotation libre.

2. - Dispositif (1) selon la revendication 1, **caractérisé par le fait que** l'ensemble bras (4) comprend au moins deux bras manipulateurs articulés entre eux, à savoir au moins un bras manipulateur proximal (40) articulé au mât (3) et un bras manipulateur distal (42) articulé au bras manipulateur proximal (40) par une liaison pivot libre d'axe parallèle à l'axe longitudinal (X1) du mât (3) et portant l'ensemble rampe de décontamination (5).

3. - Dispositif (1) selon la revendication 2, **caractérisé par le fait que** l'ensemble bras (4) comprend un bras manipulateur intermédiaire (41) articulé entre le bras manipulateur proximal (40) et le bras manipulateur distal (42) par des liaisons pivot libre d'axes (A2, A3) parallèles à l'axe longitudinal (X1) du mât (3).

4. - Dispositif (1) selon l'une quelconque des revendications 2 et 3, **caractérisé par le fait que** la liaison pivot libre entre deux bras manipulateurs articulés entre eux est configurée pour autoriser le pivotement des bras entre une position rabattue, dans laquelle les axes longitudinaux des bras forment un angle nul ou quasi-nul, et une position d'utilisation, dans laquelle les axes longitudinaux des bras forment des angles non nuls.

5. - Dispositif (1) selon l'une quelconque des revendications 1 à 4, **caractérisé par le fait que** l'ensemble rampe de décontamination (5) comprend un bras porte-rampe (50) portant la rampe (51), le bras porte-rampe (50) s'étendant selon un axe parallèle à l'axe longitudinal (X1) du mât (3), la liaison pivot libre entre l'ensemble bras (4) et l'ensemble rampe de décontamination (5) étant une liaison pivot autour de l'axe longitudinal du bras porte-rampe (50), la perche de commande (6) étant reliée solidaire en rotation au bras porte-rampe (50) et s'étendant selon un axe coaxial à l'axe longitudinal du bras porte-rampe (50).

6. Dispositif (1) selon la revendication 5, **caractérisé par le fait que** la rampe (51) est montée mobile en translation le long du bras porte-rampe (50) par une liaison glissière avec le bras porte-rampe (50).

7. - Dispositif (1) selon l'une quelconque des revendications 5 et 6, **caractérisé par le fait que** la rampe (51) est montée mobile en rotation autour d'un axe de rotation qui est perpendiculaire à la fois à l'axe longitudinal du bras porte-rampe (50) et à un axe longitudinal de la rampe (51), et/ou autour de son axe longitudinal.

8. - Dispositif (1) selon l'une quelconque des revendications 1 à 7, **caractérisé par le fait qu'**il comprend en outre une unité de surveillance (7) comprenant au moins une caméra (70) agencée de manière à être apte à viser une zone (Z) vers laquelle sont tournées les buses de diffusion (52) et un écran (71) apte à afficher l'image de la caméra (70).

9. - Dispositif (1) selon l'une quelconque des revendications 1 à 8, **caractérisé par le fait que** la perche de commande (6) est montée de manière télescopique dans l'ensemble rampe de décontamination (5), le cas échéant dans le bras porte-rampe (50).

10. - Dispositif (1) selon l'une quelconque des revendications 1 à 9, **caractérisé par le fait que** la première extrémité (3a) du mât (3) est articulée sur une embase (30) par un axe de pivotement orthogonal à l'axe longitudinal (X1) du mât (3), un actionneur (31) couplé au mât (3) et destiné à être fixé à une structure support à laquelle est assujettie l'embase (30) permettant un mouvement de pivotement du mât (3) autour dudit axe de pivotement entre une position d'utilisation verticale et une position de rangement horizontale.

11. - Véhicule de décontamination (2) de surfaces en hauteur, pour décontaminer notamment une surface de toit (S) d'un véhicule militaire (V) soumis à une contamination, le véhicule (2) étant **caractérisé par le fait qu'**il comprend :
- un châssis mobile (20) ;
- une unité (8) d'alimentation en produit décontaminant supportée par le châssis mobile (20) ; et
- un dispositif de décontamination (1) selon l'une quelconque des revendications 1 à 9, le mât (3) étant assujetti audit châssis mobile (20) ou à ladite unité (8) d'alimentation en produit décontaminant, la rampe (51) étant reliée fluidiquement à l'unité (8) d'alimentation en produit décontaminant par un chemin fluidique (9) s'étendant le long du mât (3), puis le long de l'ensemble bras (4).

## Patentansprüche

1. - Dekontaminationsvorrichtung (1) für Oberflächen in der Höhe, insbesondere zum Dekontaminieren einer Dachfläche (S) eines einer Kontamination ausgesetzten militärischen Fahrzeugs (V), wobei die Vorrichtung (1) umfasst:
- einen Mast (3), der sich gemäß einer Längsachse (X1) erstreckt und ein erstes Ende (3a) aufweist, das dazu bestimmt ist, an einer Trägerstruktur befestigt zu werden, und ein gegenüberliegendes zweites Ende (3b);
- eine Arm-Anordnung (4), die an dem zweiten Ende (3b) des Masts (3) senkrecht zu der Längsachse (X1) des Masts (3) auskragend angebracht ist und frei drehbar um die Längsachse (X1) des Masts (3) angebracht ist;
- eine Dekontaminationsrampen-Anordnung (5), die an dem auskragenden Ende der Arm-Anordnung (4) angebracht und frei drehbar um eine Achse (A4) angebracht ist, die parallel zu der Längsachse (X1) des Masts (3) ist, wobei die Dekontaminationsrampen-Anordnung (5) mindestens eine Rampe (51) umfasst, die dazu bestimmt ist, mit einer Versorgungseinheit (8) für Dekontaminationsmittel verbunden zu werden, wobei sich die mindestens eine Rampe (51) senkrecht zu der Längsachse (X1) des Masts (3) erstreckt und mit Diffusionsdüsen (52) versehen ist, die auf einer Seite der Rampe (51) angeordnet sind;
wobei die Vorrichtung (1) **dadurch gekennzeichnet ist, dass** sie ferner eine Bedienungsstange (6) umfasst, die so konfiguriert ist, dass sie für einen Bediener (O) am Boden zugänglich ist und mit der Dekontaminationsrampen-Anordnung (5) derart gekoppelt ist, dass eine Betätigung der Bedienungsstange (6) durch einen Bediener (O) am Boden das Steuern der Drehungen der Arm-Anordnung (4) und der Dekontaminationsrampen-Anordnung (5) um ihre freien Drehachsen (A1-A4) ermöglicht.

2. - Vorrichtung (1) nach Anspruch 1, **dadurch gekennzeichnet, dass** die Arm-Anordnung (4) mindestens zwei gelenkig miteinander verbundene Betätigungsarme umfasst, nämlich mindestens einen proximalen Betätigungsarm (40), der an dem Mast (3) angelenkt ist, und einen distalen Betätigungsarm (42), der an dem proximalen Betätigungsarm (40) durch eine freie Schwenkverbindung mit einer Achse, die parallel zu der Längsachse (X1) des Masts (3) ist, angelenkt ist und die Dekontaminationsrampen-Anordnung (5) trägt.

3. - Vorrichtung (1) nach Anspruch 2, **dadurch gekennzeichnet, dass** die Arm-Anordnung (4) einen zwischen dem proximalen Betätigungsarm (40) und dem distalen Betätigungsarm (42) durch freie Schwenkverbindungen mit Achsen (A2, A3), die parallel zu der Längsachse (X1) des Masts (3) sind, angelenkten zwischengestellten Betätigungsarm (41) umfasst.

4. - Vorrichtung (1) nach einem der Ansprüche 2 und 3, **dadurch gekennzeichnet, dass** die freie Schwenkverbindung zwischen zwei aneinander angelenkten Betätigungsarmen so konfiguriert ist, dass sie das Schwenken der Arme zwischen einer eingeklappten Stellung, in welcher die Längsachsen der Arme einen Winkel bilden, der Null oder nahezu Null ist, und einer Gebrauchsstellung, in welcher die Längsachsen der Arme von Null verschiedene Winkel bilden, gestattet.

5. - Vorrichtung (1) nach einem der Ansprüche 1 bis 4, **dadurch gekennzeichnet, dass** die Dekontaminationsrampen-Anordnung (5) einen Rampenträgerarm (50) umfasst, der die Rampe (51) trägt, wobei sich der Rampenträgerarm (50) gemäß einer Achse erstreckt, die parallel zu der Längsachse (X1) des Masts (3) ist, wobei die freie Schwenkverbindung zwischen der Arm-Anordnung (4) und der Dekontaminationsrampen-Anordnung (5) eine Schwenkverbindung um die Längsachse des Rampenträgerarms (50) ist, wobei die Bedienungsstange (6) drehfest mit dem Rampenträgerarm (50) verbunden ist und sich gemäß einer Achse erstreckt, die koaxial zu der Längsachse des Rampenträgerarms (50) ist.

6. - Vorrichtung (1) nach Anspruch 5, **dadurch gekennzeichnet, dass** die Rampe (51) entlang des Rampenträgerarms (50) durch eine Gleitverbindung mit dem Rampenträgerarm (50) translatorisch bewegbar angebracht ist.

7. - Vorrichtung (1) nach einem der Ansprüche 5 und 6, **dadurch gekennzeichnet, dass** die Rampe (51) drehbeweglich um eine Drehachse angebracht ist, die sowohl zu der Längsachse des Rampenträgerarms (50) als auch zu einer Längsachse der Rampe (51) senkrecht ist, und/oder um ihre Längsachse.

8. - Vorrichtung (1) nach einem der Ansprüche 1 bis 7, **dadurch gekennzeichnet, dass** sie ferner eine Überwachungseinheit (7) umfasst, die mindestens eine Kamera (70) umfasst, die so angeordnet ist, dass sie geeignet ist, auf einen Bereich (Z) gerichtet zu sein, zu dem die Diffusionsdüsen (52) gerichtet sind, und einen Bildschirm (71), der dazu geeignet ist, das Bild der Kamera (70) anzuzeigen.

9. - Vorrichtung (1) nach einem der Ansprüche 1 bis 8, **dadurch gekennzeichnet, dass** die Bedienungsstange (6) teleskopisch in der Dekontaminationsrampen-Anordnung (5), gegebenenfalls in dem Rampenträgerarm (50), angebracht ist.

10. - Vorrichtung (1) nach einem der Ansprüche 1 bis 9, **dadurch gekennzeichnet, dass** das erste Ende (3a) des Masts (3) an einem Sockel (30) durch eine Schwenkachse angelenkt ist, die orthogonal zu der Längsachse (X1) des Masts (3) ist, wobei ein an den Mast (3) gekoppelter und dazu bestimmter Aktuator (31), an einer Trägerstruktur befestigt zu werden, an welcher der Sockel (30) befestigt ist, eine Schwenkbewegung des Masts (3) um die Schwenkachse zwischen einer vertikalen Gebrauchsstellung und einer horizontalen Lagerstellung ermöglicht.

11. - Dekontaminationsfahrzeug (2) für Oberflächen in der Höhe, insbesondere zum Dekontaminieren einer Dachfläche (S) eines einer Kontamination ausgesetzten militärischen Fahrzeugs (V), wobei das Fahrzeug (2) **dadurch gekennzeichnet ist, dass** es umfasst:
- ein mobiles Fahrgestell (20);
- eine von dem mobilen Fahrgestell (20) getragene Versorgungseinheit (8) für Dekontaminationsmittel; und
- eine Dekontaminationsvorrichtung (1) nach einem der Ansprüche 1 bis 9, wobei der Mast (3) an dem mobilen Fahrgestell (20) oder an der Versorgungseinheit (8) für Dekontaminationsmittel befestigt ist, wobei die Rampe (51) mit der Versorgungseinheit (8) für Dekontaminationsmittel durch einen Fluidweg (9), der sich entlang des Masts (3), dann entlang der Arm-Anordnung (4) erstreckt, fluidisch verbunden ist.

## Claims

1. - A device (1) for decontaminating surfaces at height, for decontaminating notably a roof surface (S) of a military vehicle (V) subjected to contamination, which device (1) comprises:
- a mast (3) extending according to a longitudinal axis (X1) and having a first end (3a) intended to be fixed to a support structure and a second end (3b) opposite;
- an arm assembly (4) mounted in cantilever manner at the second end (3b) of the mast (3) perpendicular to the longitudinal axis (X1) of the mast (3), and mounted for free rotation about the longitudinal axis (X1) of the mast (3);
- a decontamination spray bar assembly (5) mounted at the cantilevered end of the arm assembly (4) and for free rotation about an axis (A4) parallel to the longitudinal axis (X1) of the mast (3), the decontamination spray bar assembly (5) comprising at least one spray bar (51) intended to be connected to a unit (8) for supplying a decontamination product, said at least one spray bar (51) extending perpendicular to the longitudinal axis (X1) of the mast (3) and being provided with diffusion nozzles (52) arranged on one face of the spray bar (51);
the device (1) being **characterized in that** it further comprises a control pole (6) configured to be accessible by an operator (O) on the ground and coupled to the decontamination spray bar assembly (5) in such a way that handling of the control pole (6) by an operator (O) on the ground allows to control the rotations of the arm assembly (4) and of the decontamination spray bar assembly (5) about their free-rotation axes (A1-A4).

2. - The device (1) according to claim 1, **characterized in that** the arm assembly (4) comprises at least two manipulator arms articulated to one another, namely at least one proximal manipulator arm (40) articulated to the mast (3) and one distal manipulator arm (42) articulated to the proximal manipulator arm (40) by a free pivot connection of axis parallel to the longitudinal axis (X1) of the mast (3) and carrying the decontamination spray bar assembly (5).

3. - The device (1) according to claim 2, **characterized in that** the arm assembly (4) comprises an intermediate manipulator arm (41) articulated between the proximal manipulator arm (40) and the distal manipulator arm (42) by free pivot connections of the axes (A2, A3) parallel to the longitudinal axis (X1) of the mast (3).

4. - The device (1) according to any one of claims 2 and 3, **characterized in that** the free pivot connection between two manipulator arms articulated to one another is configured to allow pivoting of the arms between a folded position, in which the longitudinal axes of the arms form a zero or quasi-zero angle, and a use position, in which the longitudinal axes of the arms form non-zero angles.

5. - The device (1) according to any one of claims 1 to 4, **characterized in that** the decontamination spray bar assembly (5) comprises a spray bar support arm (50) carrying the spray bar (51), the spray bar support arm (50) extending according to an axis parallel to the longitudinal axis (X1) of the mast (3), the free pivot connection between the arm assembly (4) and the decontamination spray bar assembly (5) being a pivot connection about the longitudinal axis of the spray bar support arm (50), the control pole (6) being connected integral in rotation with the spray bar support arm (50) and extending according to an axis coaxial with the longitudinal axis of the spray bar support arm (50).

6. - The device (1) according to claim 5, **characterized in that** the spray bar (51) is mounted movable in translation along the spray bar support arm (50) by a sliding connection with the spray bar support arm (50).

7. - The device (1) according to any one of claims 5 and 6, **characterized in that** the spray bar (51) is mounted movable in rotation about a rotation axis which is perpendicular both to the longitudinal axis of the spray bar support arm (50) and to a longitudinal axis of the spray bar (51), and/or about its longitudinal axis.

8. - The device (1) according to any one of claims 1 to 7, **characterized in that** it further comprises a monitoring unit (7) comprising at least one camera (70) arranged so as to be able to aim at a zone (Z) toward which the diffusion nozzles (52) are turned and a screen (71) able to display the image from the camera (70).

9. - The device (1) according to any one of claims 1 to 8, **characterized in that** the control pole (6) is mounted in a telescopic manner in the decontamination spray bar assembly (5), where appropriate in the spray bar support arm (50).

10. - The device (1) according to any one of claims 1 to 9, **characterized in that** the first end (3a) of the mast (3) is articulated on a base (30) by a pivot axis orthogonal to the longitudinal axis (X1) of the mast (3), an actuator (31) coupled to the mast (3) and intended to be fixed to a support structure to which the base (30) is fastened allowing a pivoting movement of the mast (3) about said pivot axis between a vertical use position and a horizontal storage position.

11. - A decontamination vehicle (2) for surfaces at height, for decontaminating notably a roof surface (S) of a military vehicle (V) subjected to contamination, the vehicle (2) being **characterized in that** it comprises:
- a mobile chassis (20);
- a unit (8) for supplying a decontamination product supported by the mobile chassis (20); and
- a decontamination device (1) according to any one of claims 1 to 9, the mast (3) being fastened to said mobile chassis (20) or to said unit (8) for supplying a decontamination product, the spray bar (51) being fluidly connected to the unit (8) for supplying a decontamination product by a fluid path (9) extending along the mast (3), then along the arm assembly (4).
